# EUROPEAN PATENT APPLICATION

(11) **EP 2 098 191 A1**
(43) Date of publication of application: **09.09.2009**
(21) Application number: 09154466.8
(22) Date of filing: 05.03.2009
(51) Int. Cl.: A61F 2/06, A61B 17/12, A61F 2/00, A61F 2/82

(54) **Aneurysm shield anchoring device**

(30) Priority: 05.03.2008 US 42382
(71) Applicant: Neurovasx, Inc., Maple Grove, MN 55369 (US)
(72) Inventor: Lee, Jeffrey, A., Plymouth, MN 55447 (US)
(74) Representative: Curley, Donnacha John

(57) **Abstract**

An aneurysm shield anchoring device includes a curved surface to cover an aneurysm. The curved surface includes a major axis with first and second major-axis edges and a minor axis orthogonal to the major axis with first and second minor-axis edges. The curved surface includes an eccentric form factor disposed symmetrically along the major axis as well as a semi-circular form factor orthogonal to the major axis. A spring is coupled to the curved surface such that the spring is disposed to urge more force upon the curved surface at the minor-axis edges than at the major-axis edges. The curved surface is also configured with an aneurysm-occlusion structure.

## Description

### TECHNICAL FIELD

Inventive subject matter described herein relates to aneurysm shield embodiments, aneurysm shield anchoring mechanism embodiments and method embodiments for making and using aneurysm shields and aneurysm shield anchoring mechanisms, referred to as an aneurysm treatment system.

### COPYRIGHT

A portion of the disclosure of this patent document contains material that is subject to copyright protection. The copyright owner has no objection to the facsimile reproduction by anyone of the patent document or the patent disclosure, as it appears in the Patent and Trademark Office patent files or records, but otherwise reserves all copyright rights whatsoever. This notice applies to the products, processes and data as described below and in the tables that form a part of this document: Copyright 2008, Neurovasx, Inc. All Rights Reserved.

### BACKGROUND

An aneurysm is a balloon-like swelling in a wall of a blood vessel. An aneurysm results in weakness of the vessel wall in which it occurs. This weakness predisposes the vessel to tear or rupture with potentially catastrophic consequences for any individual having the aneurysm. Vascular aneurysms are a result of an abnormal dilation of a blood vessel, usually resulting from disease and/or genetic predisposition, which can weaken the arterial wall and allow it to expand. Aneurysm sites tend to be areas of mechanical stress concentration so that fluid flow seems to be the most likely initiating cause for the formation of these aneurysms.

Aneurysms in cerebral circulation tend to occur in an anterior communicating artery, a posterior communicating artery, or a middle cerebral artery. The majority of these aneurysms arise either from curvature in the vessels or at bifurcations of these vessels. Cerebral aneurysms are most often diagnosed by the rupture and subarachnoid bleeding of the aneurysm.

Cerebral aneurysms are most commonly treated in open surgical procedures where the diseased vessel segment is clipped across the base of the aneurysm. While considered to be an effective surgical technique, particularly considering an alternative which may be a ruptured or re-bleed of a cerebral aneurysm, conventional neurosurgery suffers from a number of disadvantages. The surgical procedure is complex and requires experienced surgeons and well-equipped surgical facilities. Current treatment options for cerebral aneurysm fall into two categories, surgical and interventional.

### BRIEF DESCRIPTION OF THE DRAWINGS

In order to depict the manner in which the embodiments are obtained, a more particular description of embodiments briefly described above will be rendered by reference to exemplary embodiments that are illustrated in the appended drawings. Understanding that these drawings depict only typical embodiments that are not necessarily drawn to scale and are not, therefore, to be considered to be limiting of its scope, the embodiments will be described and explained with additional specificity and detail through the use of the accompanying drawings in which:
FIG. 1 is a top plan of an aneurysm treatment system according to an embodiment;
FIG. 2 is a cross-section elevation of the aneurysm treatment system depicted in FIG. 1 according to an embodiment;
FIG. 3 is an elevational perspective of the aneurysm treatment system depicted in FIG. 1 according to an embodiment;
FIGs. 4a and 4b are front elevations of the aneurysm treatment system depicted in FIG. 1 according to an embodiment;
FIG. 5 is an elevational perspective of the aneurysm treatment system according to an embodiment;
FIG. 6 is a side elevation of the aneurysm treatment system depicted in FIG. 5 according to an embodiment;
FIG. 7 is a cut-away elevation of an aneurysm treatment system when it is deployed within a bio lumen according to an embodiment;
FIG. 8 is a top plan of an aneurysm treatment system according to an embodiment;
FIG. 9 is a front elevation of an aneurysm treatment system according to an embodiment;
FIG. 10 is a perspective elevation of an aneurysm treatment system according to an embodiment; and
FIG. 11 is a cut-away elevation of an aneurysm treatment system when it is deployed within a bio lumen according to an embodiment; and
FIG. 12 is a method flow diagram deploying an aneurysm treatment system according to an embodiment.

### DETAILED DESCRIPTION

Although detailed embodiments are disclosed herein, it is to be understood that the disclosed embodiments are merely exemplary of the embodiments that may be configured in various and alternative forms. Specific structural and functional details disclosed herein are not to be interpreted as limiting, but merely as a basis for teaching one skilled in the art to variously employ the aneurysm treatment system embodiments. Throughout the drawings, like elements may be given with like numerals.

Referred to herein are trade names for materials including, but not limited to, polymers and optional components. Reference to such trade names is not intended to limit such materials described and referenced by a certain trade name. Equivalent materials (e.g., those obtained from a different source under a different name or catalog (reference) number to those referenced by trade name may be substituted and utilized in the methods described and claimed herein. All percentages and ratios are calculated by weight unless otherwise indicated. All percentages are calculated based on the total composition unless otherwise indicated. All component or composition levels are in reference to the active level of that component or composition, and are exclusive of impurities, for example, residual solvents or by-products, which may be present in commercially available sources.

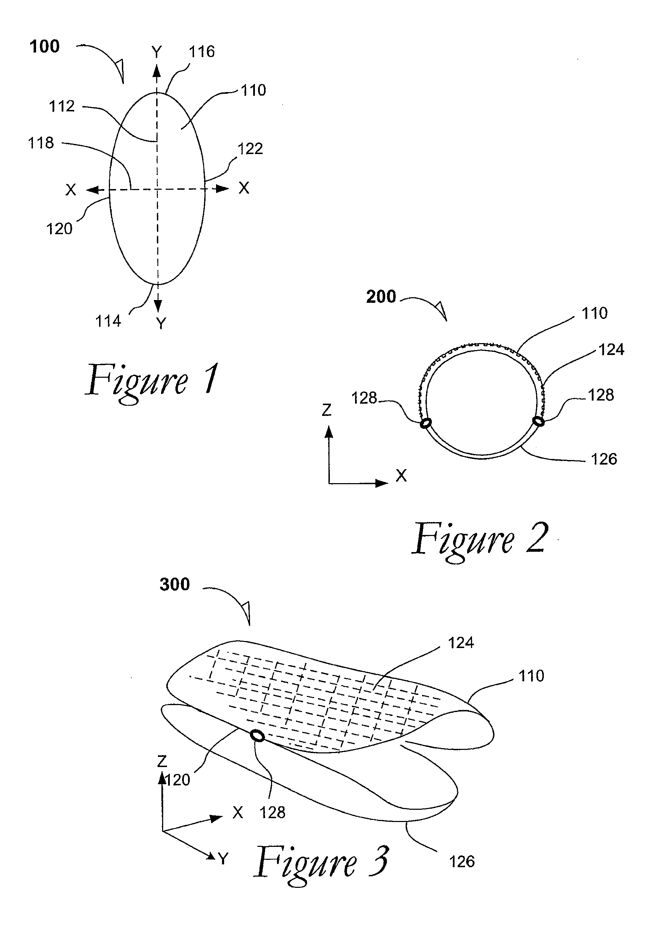

FIG. 1 is a top plan of an aneurysm treatment system 100 according to an embodiment. The aneurysm treatment system 100 may be referred to as a device 100 for occluding an aneurysm. The aneurysm treatment system 100 may be referred to as an aneurysm shield anchoring device 100.

The aneurysm treatment system 100 includes a curved surface 110 to shield an aneurysm. The curved surface 110 may also be referred to as an aneurysm shield 110. The curved surface 110 includes a major (or longitudinal) axis 112 with first and second major-axis edges 114 and 116 respectively. The aneurysm treatment system 100 also includes a minor axis 118 that is orthogonal to the major axis 112. The aneurysm treatment system 100 also includes first and second minor-axis edges 120 and 122, respectively. In an embodiment, the aneurysm treatment system 100 exhibits an eccentric form factor that is disposed symmetrically along the axes 112 and 118.

FIG. 2 is an elevational cross section 200 of the aneurysm treatment system 100 depicted in FIG. 1. In the cross-section elevation, the curved surface 110 exhibits a semi-circular form factor that is orthogonal to the major axis 112. In FIG. 2, the major axis 112 is depicted as orthogonal to the plane of the FIG. An aneurysm-occlusion structure 124 is disposed along the curved surface 110. The aneurysm-occlusion structure 124 is depicted in arbitrary shape and frequency. In an embodiment, the aneurysm-occlusion structure 124 is a plurality of seats for cell-growth materials. In an embodiment, the aneurysm-occlusion structure 124 is a plurality of seats to anchor expandable materials. A spring 126 completes a circular structure that includes the curved surface 110. The spring 126 and the curved surface 110 comprise an aneurysm shield anchoring device.

FIG. 3 is an elevational perspective 300 of the aneurysm treatment system 100 depicted in FIG. 1 according to an embodiment. In an embodiment, the curved surface 110 carries the aneurysm-occlusion structure 124, and the curved surface 110 is configured such that insertion of the aneurysm treatment system 300 into a bio lumen will allow the aneurysm-occlusion structure 124 to be urged against an aneurysm when it is deployed.

In an embodiment, the aneurysm treatment system 300 includes the spring 126 that is coupled to the curved surface 110. As illustrated, the spring 126 is connected by an affixture 128 at the minor-axis edges 120 and 122. The affixture 128 is depicted in FIG. 3 as a local point structure, such as a staple or rivet. In an embodiment, the affixture 128 is a heat weld between the curved surface 110 and the spring 126. In an embodiment, the affixture 128 is an adhesive material between the curved surface 110 and the spring 126. Other structures for the affixture 128 may be used according to conventional technique. When viewed in elevational cross section as in FIG. 2, the spring 126 completes the formation of a circular structure with the curved surface 110 as the remainder of the circular structure.

In an embodiment, the spring 126 is made of one or more of wires. Usable wire materials include super elastic Nitinol, NIP35N, Beta 3 titanium, stainless steel, and shape memory Nitinol. It is believed that any plastically deformable, biocompatible material such as a metal, alloy or polymer is suitable for use as at least a component of the spring 126.

In an embodiment, the spring 126 is constructed of "shape memory" metal alloy (e.g., Nitinol) capable of self-expansion at internal temperatures of the target vessel host. In an embodiment, the spring 126 is configured after tomographical data of the aneurysm site has been ascertained, such that upon deployment into the target bio lumen, the spring 126 may expand to urge the curved surface 110 against the ascertained topology of the aneurysm site. Consequently, a programmed self-expansion temperature of the spring 126 may be installed into the aneurysm treatment system 100

The manner by which the spring 126 is manufactured is not particularly restricted. In an embodiment, the spring 126 is produced by laser cutting techniques applied to a starting material. Thus, the starting material could be a thin tube or sheet of a metal, alloy or polymer as described above. In an embodiment, the spring 126 is cut from a shape memory metal and is contoured to reach a final shape under temperature conditions of the target vessel for which the aneurysm treatment system 100 is to be deployed.

In an embodiment, the aneurysm-occlusion structure 124 includes a mesh material that facilitates occlusion of an aneurysm. Such aneurysm-occlusion mesh material may have a pore size that facilitates *in vivo* fibrotic cell growth in the aneurysm-occlusion structure 124. In an example embodiment, the aneurysm-occlusion structure 124 includes a polymeric material such as polyethylene.

In an embodiment, the aneurysm-occlusion structure 124 includes a hydrogel foam portion that can be entrained in a polyethylene matrix. In an embodiment, the hydrogel is swellable and has a swell ratio of 10:1-2:1. The foam provides a desirable surface for rapid cell ingrowth. The hydrogel foam or other filler material is shapable at the aneurysm neck to form a smooth, closed surface at the aneurysm neck.

Swellable materials for use as the aneurysm-occlusion structure 124 include acrylic-based materials according to an embodiment. For example, the aneurysm-occlusion structure 124 is deployed upon the curved surface 110, where the curved surface 110 is a core material that is stiffer than the outer material of the aneurysm-occlusion structure 124. In an embodiment, at least some active portions of the aneurysm-occlusion structure 124 have a time-dependent rate of dissolution such that swellable materials are encapsulated, but after deployment in a target vessel, the swellable materials may be contacted with *in vivo* fluids that dissolve encapsulation layer(s).
The hydrogel deployed within the aneurysm-occlusion structure 124 may be stiffened as a consequence of an increased degree of crosslinkage as compared to the an outer layer of the aneurysm-occlusion structure 124. While a hydrogel is described, it is understood that other biocompatible, swellable materials are suitable for use in selected embodiments. Other materials include acetates, such as cellulose acetate or polyvinylacetate, for the aneurysm-occlusion structure 124. Other materials include alcohols, such as ethylene vinyl alcohol copolymers, for the aneurysm-occlusion structure 124. Other materials include nitriles, such as polyacrylonitriles, for the aneurysm-occlusion structure 124. Other materials include cellulose acetate butyrate, nitrocellulose, copolymers ofurethane/carbonate, copolymers of styrene/maleic acid, or mixtures thereof for the aneurysm-occlusion structure 124. In particular, a hydrogel/polyurethane foam is usable in for the aneurysm-occlusion structure 124.

In an embodiment, the aneurysm-occlusion structure 124 includes a polymer-based, coil-like structure that is fabricated with soft biocompatible polymers such as PTFE, urethanes, polyolefins, nylons and the like. In an embodiment, the aneurysm-occlusion structure 124 includes at least one of solid strings and hollow strings. The aneurysm-occlusion structure 124 may also be fabricated from biodegradable materials such as PLA, PGA, PLGA, polyanhydrides and other similar biodegradable materials. Use of biodegradable materials provokes a wound healing response and concomitantly eliminates a mass effect of the aneurysm shield anchor over time.

The material of the aneurysm-occlusion structure 124 described herein may be one or more of polymeric and polymeric hybrids such as PEBAX, Grilamids, polyester, and silica. Aneurysm-occlusion structure materials may also include reabsorbables such as PGLA, PEG, PGLA and base polymer. Other aneurysm-occlusion structure materials may include textiles such as rayon, nylon, silk, Kyeon, Kevlar, and cotton. Other aneurysm-occlusion structure materials may include biopolymers such as collagen, filaments, and coated polymeric material. Other aneurysm-occlusion structure materials may include elastomers such as urethanes, silicones, nitriles, TecoFlex® of Thermedics, Inc. of Woburn, Massachusetts, Carbothane® of Noveon IP Holding Corp. of Cleveland Ohio, and silicone hybrids.

In an embodiment, the aneurysm treatment system 100 may include a coating material thereon. The coating material may be disposed continuously or discontinuously on the surface of the aneurysm treatment system 100, including both the curved surface 110 and the spring 126. The coating may be disposed on the interior and/or the exterior surface(s) of the aneurysm treatment system 100. The coating material can be one or more of a biologically inert material (e.g., to reduce the thrombogenicity of the prosthesis), a medicinal composition which leaches into the wall of the body passageway after implantation (e.g., to provide anticoagulant action, to deliver a pharmaceutical to the body passageway) and the like.

For some embodiments, the aneurysm treatment system 100 is provided with a biocompatible coating in order to minimize adverse interaction with the walls of the body vessel and/or with the liquid, usually blood, flowing through the vessel. For some embodiments, the coating is a polymeric material, which is provided by applying to the prosthesis a solution or dispersion of preformed polymer in a solvent and removing the solvent. Non-polymeric coating material may alternatively be used. Suitable coating materials, for instance polymers, may be polytetraflouroethylene or silicone rubbers, or polyurethanes that are known to be biocompatible. In an embodiment the polymer has zwitterionic pendant groups, generally ammonium phosphate ester groups, such as phosphoryl choline groups or analogues thereof.

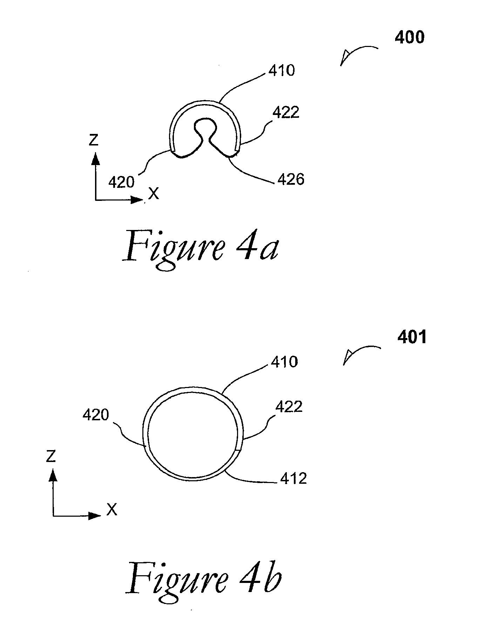

FIG. 4a is a front cross-section elevation of an aneurysm treatment system 400 according to an embodiment. Minor-axis edges 420 and 422 respectively, are depicted similar to the minor-axis edges 120 and 122 depicted in FIG. 1. In an embodiment, deployment of the aneurysm treatment system 400 includes first folding the spring 426 to decrease the X-Z dimensional footprint. By folding the spring 426, the aneurysm treatment system 400 may be inserted into a bio lumen, followed by releasing the spring from the folded configuration. In an embodiment, the spring 426 is a shape-memory material such as a shape-memory alloy. In an embodiment, the spring 426 is a shape-memory material such as a biased plastic.

FIG. 4b is a front elevation of the aneurysm treatment system depicted in FIG. 4a after further deployment. The spring 426 (FIG. 4a) in the aneurysm treatment system 401 has been released from the folded configuration, such that the aneurysm treatment system 401 has been allowed to alter the shape of the curved surface 410, such that the curved surface 410 may be urged against the wall of a bio lumen to occlude an aneurysm.

In this manner of deployment, the spring 426 is disposed to urge more force upon the curved surface 410 at the minor-axis edges 420 and 422, respectively, than at the major-axis edges. Accordingly, the method allows for a customized configuration of at least a portion of the spring 426 to achieve an altered shape of the curved surface 410. This altered shape may conform to a unique concave surface within a bio lumen.

It can now be seen that method embodiments of allowing the curved surface, e.g., curved surface 410, to alter shape to be urged against and occlude an aneurysm, may be carried out by methods other than folding the spring 426. Other methods of folding the aneurysm treatment system 401, or a part thereof, may be employed such that the aneurysm treatment system 401 may be allowed to alter the shape of the curved surface within a bio lumen.

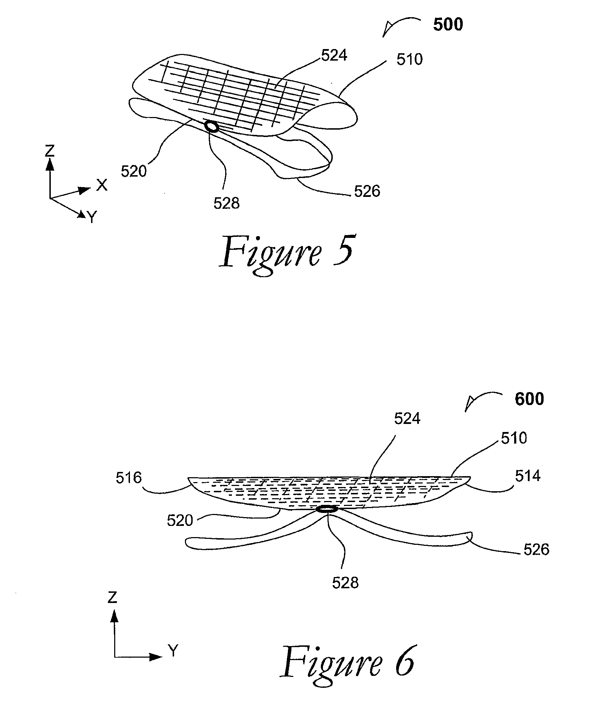

FIG. 5 is an elevational perspective of an aneurysm treatment system 500 according to an embodiment. A curved surface 510 carries the aneurysm-occlusion structure 524 such that insertion of the aneurysm treatment system 500 into a bio lumen will allow the aneurysm-occlusion structure 524 to alter shape and for the curved surface 510 to be urged against an aneurysm when it is deployed.

In an embodiment, the aneurysm treatment system 500 includes a spring 526 that is coupled to the curved surface 510. The spring 526 exhibits a racetrack form factor. As illustrated, the spring 526 is connected by an affixture 528 at the minor-axis edges 520 and 522. The affixture 528 may be any embodiment as set forth in this disclosure.

FIG. 6 is a side elevation of the aneurysm treatment system depicted in FIG. 5. An aneurysm treatment system 600 is illustrated with a longitudinal (Y-axis) dimension running across the plane of the figure, and the minor axis (X) running into and out of the plane of the figure. The spring 526 is illustrated as providing foot regions at the ends thereof that are below first and second major-axis edges 514 and 516 respectively. This embodiment illustrates a smaller material-volume presence that the aneurysm treatment system depicted in FIGs. 1-4b. When the aneurysm treatment system 600 is deployed within a bio lumen, it may have less fluid-flow hindrance below the curved surface 510 than below the curved surface 110 depicted in the previous FIGs.

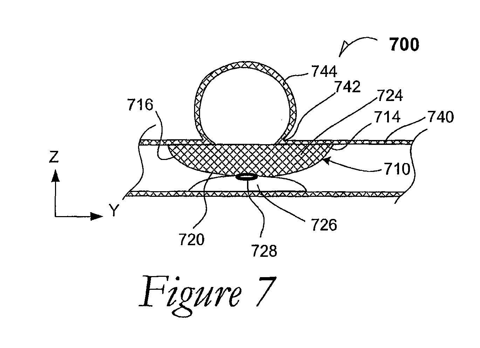

FIG. 7 is a cut-away elevation of an aneurysm treatment system 700 when it is deployed within a bio lumen according to an embodiment. A bio lumen 740 is depicted with an aneurysm neck 742 and an aneurysm 744 that is distended outside the normal confines of the bio lumen 740. An aneurysm treatment system, such as the aneurysm treatment system depicted in FIGs. 1, 2, and 3 is deployed. A curved surface 710 is urged against the bio lumen 740 by use of a spring 726 and an affixture 728 that couples the curved surface 710 to the spring 726. The curved surface 710 exhibits major-axis edges 714 and 716 and a minor-axis edge 720. The spring 726 is disposed to urge more force upon the curved surface 710 at the minor-axis edges (720 depicted) than at the major-axis edges 714 and 716. Consequently, the curved surface 710 is urged against the location of the aneurysm 742 neck in the longitudinal configuration of the bio lumen 740.

An aneurysm-occlusion structure 724 is disposed along the curved surface 710. The aneurysm-occlusion structure 724 is depicted in arbitrary shape and frequency. In an embodiment, the aneurysm-occlusion structure 724 may be any aneurysm-occlusion structure set forth in this disclosure.

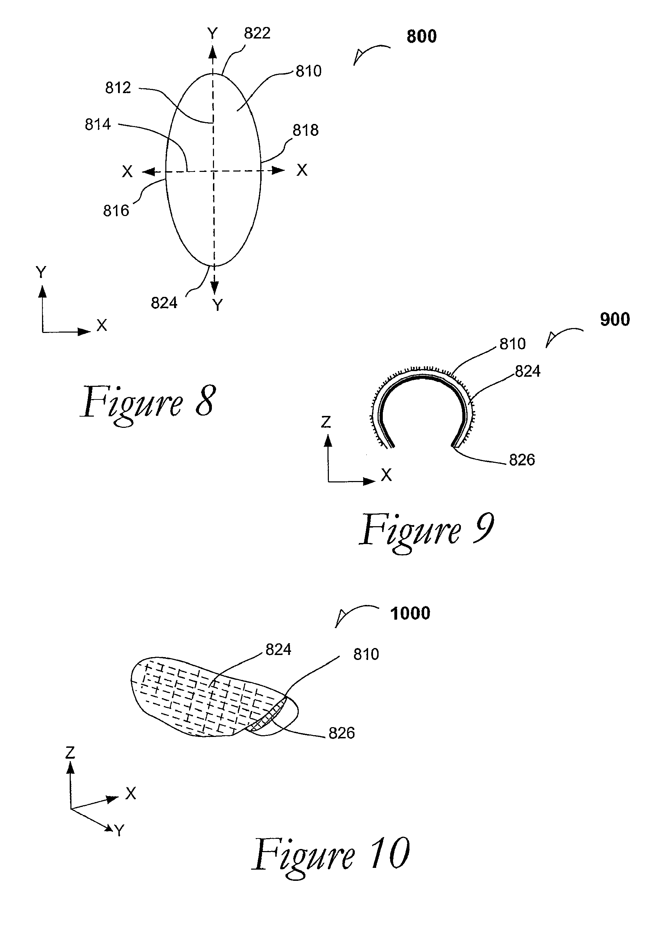

FIG. 8 is top plan of an aneurysm treatment system 800 according to an embodiment. The aneurysm treatment system 800 may be referred to as a device 100 for occluding an aneurysm. The aneurysm treatment system 800 may be referred to as an aneurysm shield anchoring device 800.

The aneurysm treatment system 800 includes a curved surface 810 to shield an aneurysm. The curved surface 810 may also be referred to as an aneurysm shield 110. The curved surface 810 has a major (or longitudinal) axis 812 with first and second major-axis edges 814 and 816 respectively. The aneurysm treatment system 800 also includes a minor axis 818 that is orthogonal to the major axis 812. The aneurysm treatment system 800 also includes first and second minor-axis edges 820 and 822, respectively. The aneurysm treatment system 800 exhibits an eccentric form factor that is disposed symmetrically along the major axis 812.

FIG. 9 is an elevational cross section 900 of the aneurysm treatment system 800 depicted in FIG. 8. In the cross-section elevation, the curved surface 810 exhibits a semi-circular form factor that is orthogonal to the major axis 812. In FIG. 9, the major axis 812 is depicted as orthogonal to the plane of the figure. An aneurysm-occlusion structure 824 is disposed along the curved surface 810. The aneurysm-occlusion structure 824 is depicted in arbitrary shape and frequency. According to an embodiment, any aneurysm-occlusion structure and/or materials that are set forth in this disclosure may be used to construct the aneurysm-occlusion structure 824.

A spring 826 is located integral with and below the curved surface 810, such that it mimics the semi-circular form factor of the curved surface 810. According to an embodiment, any spring structure and/or materials that are set forth in this disclosure may be used to construct the spring 826.

The spring 826 and the curved surface 810 comprise an aneurysm shield anchoring device. In an embodiment, the spring 826 is a shape memory material such as a metal, plastic, or metalloplastic composite that can achieve a selected topology when it has been deployed into a bio lumen under the life conditions thereof.

In an embodiment, the spring 826 is not present as a separate structure. Rather, the spring is integral with the curved surface 810, in that the curved surface has been biased during formation. Consequently, the curved surface 810 has a characteristic topology that is expandable more at the minor-axis edges 816 and 818, respectively, than at the major-axis edges. In this configuration, the curved surface 810 is urgeable against an aneurysm when it is deployed.

FIG. 10 is an elevational perspective 1000 of the aneurysm treatment system 800 and 900 depicted in FIGs. 8 and 9 according to an embodiment. The curved surface 810 carries the aneurysm-occlusion structure 824, and the curved surface 810 is configured such that insertion of the aneurysm treatment system 800 into a bio lumen will allow the aneurysm-occlusion structure 824 to be urged against an aneurysm when it is deployed.

In an embodiment, the aneurysm treatment system 800 includes the spring 826 that is coupled to the curved surface 810 as depicted in FIG. 9. The spring 826 is configured such that upon deployment in a bio lumen, the minor-axis edges 820 and 822 may urged against the bio lumen wall under more force than the major-axis edges 820 and 822.

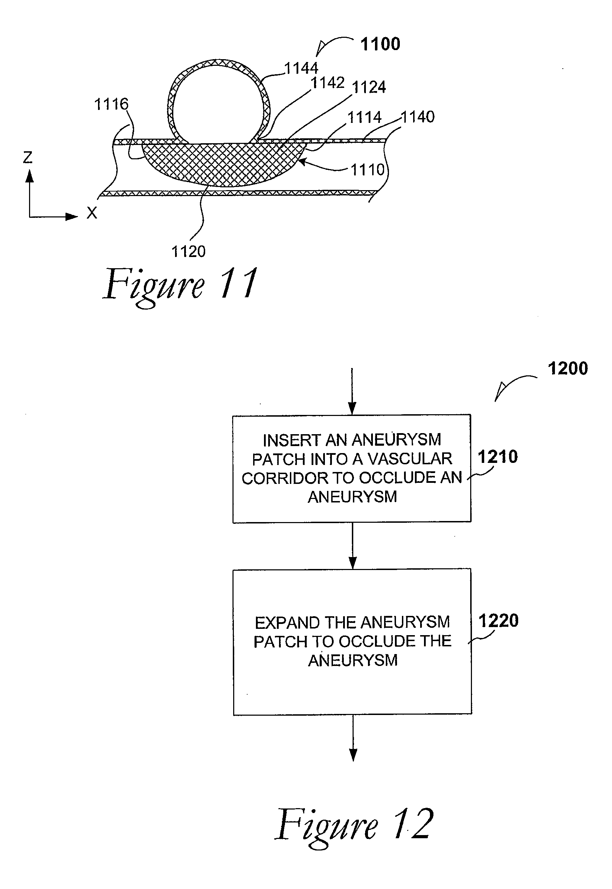

FIG. 11 is a cut-away elevation of an aneurysm treatment system 1100 when it is deployed within a bio lumen according to an embodiment. A bio lumen 1140 is depicted with an aneurysm neck 1142 and an aneurysm 1144 that is distended outside the normal confines of the bio lumen 1140. An aneurysm treatment system, such as the aneurysm treatment system depicted in FIGs. 8, 9, and 10, is deployed. A curved surface 1110 is urged against the bio lumen 1140 by use of a spring that is integral to the curved surface 1110. The curved surface 1110 exhibits major-axis edges 1114 and 1116 and a minor-axis edge 1120. A spring (obscured in FIG. 11) is disposed to urge more force upon the curved surface 1110 at the minor-axis edges (1120 depicted) than at the major-axis edges 1114 and 1116. Consequently, the curved surface 1110 is urged against the location of the aneurysm 1142 in the longitudinal configuration of the bio lumen 1140.

An aneurysm-occlusion structure 1124 is disposed along the curved surface 1110. The aneurysm-occlusion structure 1124 is depicted in arbitrary shape and frequency. In an embodiment, the aneurysm-occlusion structure 1124 may be any aneurysm-occlusion structure set forth in this disclosure.

FIG. 12 is a method flow diagram 1200 for deploying an aneurysm treatment system according to an embodiment.

At 1210, the method includes inserting an aneurysm shield anchor into a bio lumen.

At 1220, the method includes allowing the aneurysm shield anchor to assist a curved surface of the aneurysm shield anchor to alter shape to occlude an aneurysm.

The application extends to a method of deploying an aneurysm-occlusion device comprising: inserting a structure into a bio lumen at an aneurysm site, wherein the structure includes: a curved surface to cover an aneurysm, wherein the curved surface includes: a major axis with first and second major-axis edges;
a minor axis orthogonal to the major axis with first and second minor-axis edges; an eccentric form factor disposed symmetrically along the major axis; a semi-circular form factor orthogonal to the major axis; and an aneurysm-occlusion structure disposed at the curved surface; and a spring coupled to the curved surface, wherein the spring is disposed to urge more force upon the curved surface at the minor-axis edges than at the major-axis edges; and allowing the curved surface to alter shape to occlude the aneurysm. The spring may first be folded, and following inserting the structure at the aneurysm site, the spring is then unfolded to allow the curved surface to alter shape. The spring may be first configured under a first tension, and following inserting the structure at the aneurysm site, the spring is then configured under a second tension that is less than the first tension. The spring may include a race form factor, and wherein the race form factor is attached to the curved surface at the first and second minor-axis edges.

The Abstract is provided to comply with 37 C.F.R. §1.72(b) requiring an abstract that will allow the reader to quickly ascertain the nature and gist of the technical disclosure. It is submitted with the understanding that it will not be used to interpret or limit the scope or meaning of the claims.

In the foregoing Detailed Description, various features are grouped together in a single embodiment for the purpose of streamlining the disclosure. This method of disclosure is not to be interpreted as reflecting an intention that the claimed embodiments of the invention require more features than are expressly recited in each claim. Rather, as the following claims reflect, inventive subject matter lies in less than all features of a single disclosed embodiment. Thus the following claims are hereby incorporated into the Detailed Description, with each claim standing on its own as a separate preferred embodiment.

It will be readily understood to those skilled in the art that various other changes in the details, material, and arrangements of the parts and method stages, which have been described and illustrated in order to explain the nature of this invention, may be made without departing from the principles and scope of the invention as expressed in the subjoined claims.

## Claims

1. A device for occluding an aneurysm comprising:
a curved surface to cover an aneurysm, wherein the curved surface includes:
a major axis with first and second major-axis edges;
a minor axis orthogonal to the major axis with first and second minor-axis edges;
an eccentric form factor disposed symmetrically along the major axis; and
a semi-circular form factor orthogonal to the major axis;
an aneurysm-occlusion structure disposed at the curved surface; and
a spring coupled to the curved surface, wherein the spring is disposed to urge more force upon the curved surface at the minor-axis edges than at the major-axis edges.

2. The device of claim 1, wherein the spring is affixed at the minor-axis edges.

3. The device of claim 1, wherein the spring is affixed at the minor-axis edges, and wherein the spring completes a circular cross-section that includes the curved surface.

4. The device of any one of claims 1 to 3, wherein the spring forms a circular structure with the curved surface in cross section.

5. The device of claim 1, wherein the spring is integral with the curved surface.

6. The device of claim 1, wherein the spring is integral to the curved surface, and wherein the spring forms a semi-circular structure with the curved surface in cross section.

7. The device of claim 1, wherein the spring is integral with the curved surface, and wherein the spring consists of a bias within the curved surface.

8. The device of any preceding claim, wherein the aneurysm-occlusion structure includes a plurality of seats imposed in the curved surface.

9. The device of any preceding claim, wherein the aneurysm-occlusion structure includes a substance-holder disposed upon the curved surface.

10. The device of any preceding claim, wherein the aneurysm-occlusion structure includes a cell-growth factor substance.

11. The device of any preceding claim, wherein the aneurysm-occlusion structure includes an integrin substance.

12. The device of any preceding claim, wherein the aneurysm-occlusion structure includes a cell-attachment protein substance.

13. The device of device of any preceding claim, wherein the aneurysm-occlusion structure includes a cellular-growth accelerator substance.

14. The device of any preceding claim, wherein the aneurysm-occlusion structure includes a gene-containing cellular-growth accelerator substance.

15. The device of any preceding claim, wherein the aneurysm-occlusion structure includes a liquid-swellable substance.

16. The device of claim 1, wherein the spring includes a race form factor, and wherein the race form factor is attached to the curved surface at the first and second minor-axis edges.

17. The device of any preceding claim, wherein the spring and the curved surface are customized to be urged against a particular bio lumen topology.
